## Europäisches Patentamt
## European Patent Office
## Office européen des brevets

(11) Numéro de publication: **0 037 785**
**B1**

(12) **FASCICULE DE BREVET EUROPEEN**

(45) Date de publication du fascicule du brevet:
**14.08.85**

(21) Numéro de dépôt: **81400548.4**

(22) Date de dépôt: **03.04.81**

(51) Int. Cl.⁴: **A 61 F 5/44**

(54) **Dispositif atténuateur des bruits émis par un anus artificiel.**

(30) Priorité: **04.04.80 FR 8007774**

(43) Date de publication de la demande:
**14.10.81 Bulletin 81/41**

(45) Mention de la délivrance du brevet:
**14.08.85 Bulletin 85/33**

(84) Etats contractants désignés:
**AT BE CH DE GB IT LI LU NL SE**

(56) Documents cités:
**DE - B - 2 919 467**
**FR - A - 2 337 545**
**FR - A - 2 410 999**
**FR - A - 2 417 291**
**GB - A - 2 014 857**
**US - A - 2 931 353**

(73) Titulaire: **LABORATOIRES BIOTROL S.A., 1, rue du Foin, F-75140 Paris Cedex 03 (FR)**

(72) Inventeur: **Curutcharry, Jean, Lotissement Argia, Guethary F-64210 Bidart (FR)**

(74) Mandataire: **Phélip, Bruno et al, c/o Cabinet Harlé & Phélip 21, rue de la Rochefoucauld, F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Description

La présente invention concerne la suppression ou l'atténuation des bruits émis par un anus artificiel.

Les patients entérostomisés, en particulier colostomisés et iléostomisés, possèdent une ouverture intestinale débouchant dans la paroi abdominale. L'éjection des résidus intestinaux échappe le plus souvent au contrôle de ces malades.

En règle générale, le recueil des matières est effectué dans des poches en matière plastique, qui se fixent sur l'abdomen du malade par des adhésifs ou par des joints, le plus souvent en gomme naturelle ou synthétique, associés à un système de ceinture.

De tels systèmes de poches assurent effectivement la collecte des solides ou liquides dans des conditions acceptables, mais ils sont inefficaces vis-à-vis des gaz émis par le système intestinal.

Les entérostomisés pratiquant l'irrigation colique rencontrent le même problème, en particulier le deuxième jour suivant le lavement.

Ces émissions gazeuses sont imprévisibles et incontrôlables, et elles s'accompagnent généralement de bruit lors de leur passage à travers la stomie.

L'état de fait qui en résulte représente une gêne considérable pour les entérostomisés et nuit à leur réinsertion sociale.

On a certes déjà décrit des systèmes pour l'évacuation et la désodorisation des gaz émis chez les entérostomisés. Ainsi, depuis quelques temps, divers systèmes de filtres collés ou soudés sur des poches de recueil pour entérostomisés ont été proposés à cette fin (voir en particulier FR-A-2 331 549); mais, de par leur conception même, ils ne peuvent pas contribuer à éliminer les émissions sonores de l'anus artificiel et il est manifeste que cet objectif n'a pas été visé lors de leur mise au point.

On a d'autre part proposé des systèmes de bouchage, notamment magnétiques, assurant une certaine atténuation des bruits émis par une stomie sur et/ou dans laquelle un tel système de bouchage vient s'insérer (voir à ce propos, entre autres, FR-A-2 417 291 et FR-A-2 410 899, et le brevet US-A-2 931 353). Mais ces dispositifs de bouchage ne peuvent être portés à la place des poches de recueil susdites que par des entérostomisés qui s'irriguent et/ou dont les selles sont très bien contrôlées, c'est-à-dire régulières. Cela ne correspond qu'à une minorité des entérostomisés.

Il en résulte qu'aucune solution au problème de l'émission des bruits émis par une stomie n'a été proposée jusqu'à présent pour les stomisés qui utilisent des poches de recueil. Pourtant, ce type de patients représente la plus grande majorité des stomisés.

On peut même dire globalement que, jusqu'à maintenant, aucun système n'existe spécifiquement pour résoudre les problèmes posés par les émissions sonores d'un anus artificiel, et les systèmes pour l'évacuation et la désodorisation des gaz décrits jusqu'ici en association avec des poches de recueil pour stomisées ne permettent pas en eux-mêmes d'atténuer les émissions sonores provenant de l'anus artificiel sur lequel on applique ces poches.

De plus, même si certains ont cherché à associer à ces poches de recueil des dispositifs tels que ceux qui peuvent éventuellement atténuer un peu les bruits et qui font partie de certains systèmes de bouchage connus, comme indiqué plus haut, cela n'a absolument pas permis de procurer aux utilisateurs un dispositif atténuant les bruits émis par l'anus artificiel aussi bien des stomisés pratiquant l'irrigation que de ceux ne la pratiquant pas, et assurant parallèlement une bonne évacuation des selles de tous ces entérostomisés. Un dispositif atténuant les bruits émis par une stomie n'est en effet acceptable que s'il ne contrarie pas le libre passage des selles et leur recueil.

On a maintenant trouvé qu'on peut réaliser l'ensemble de ces objectifs au moyen d'un dispositif conforme à la revendication 1.

Un tel dispositif s'est en effet révélé apte à la fois à assurer une détente des gaz émis et à maintenir les parois intestinales constamment écartées; c'est par ces deux actions conjuguées qu'il peut faire disparaître toute émission sonore par la stomie. On a noté par ailleurs que ce dispositif était bénéfique pour prévenir toute sténose du stoma.

Le dispositif selon l'invention protège le patient contre les émissions sonores jusqu'à l'émission d'une selle; celle-ci s'opère normalement et a lieu après l'évacuation du dispositif mis en place dans la stomie. Le fait que ce dispositif puisse être facilement rejeté soit manuellement, soit même sans intervention extérieure autre que celle des selles, selon les modes de réalisation, constitue un atout supplémentaire.

En variante, ce dispositif peut en outre contribuer à désodoriser les gaz émis et, à cet effet, il s'est avéré très avantageux de munir les cellules du matériau poreux à cellules ouvertes d'une charge de matière désodorisante, comme par exemple du charbon actif.

L'invention a donc également pour objet un dispositif tel que décrit ci-dessus et dans lequel au moins une partie des cellules du matériau poreux à cellules ouvertes contient une charge de matière absorbante, telle que du charbon actif.

Cette charge de matière absorbante peut être introduite dans les cellules par tout moyen connu de l'homme de l'art et peut, si nécessaire, y être fixée par des moyens classiques, notamment par une résine appropriée.

L'invention est décrite ci-après en référence aux figures des planches de dessins annexées, qui l'illustrent et dans lesquelles:

Fig. 1 représente une vue schématique en coupe d'une variante de ce dispositif, également en place dans une stomie portant une poche de recueil, et associé à celle-ci.

Fig. 2 représente une vue schématique en coupe d'une variante du dispositif de la figure 1, dans laquelle la fixation du corps oblong est réalisée sur la partie frontale interne d'une poche de recueil, sur la périphérie de l'ouverture de celle-ci.

Dans le présent contexte, on entend par «corps oblong» (1) tout corps ayant une symétrie axiale de révolution et dont la longueur maximale est de 1 à 5 fois environ le diamètre maximum.

En pratique, le plus grand diamètre du corps oblong peut être de 5 à 40 mm environ, tandis que sa longueur peut être par exemple de 5 à 50 mm environ.

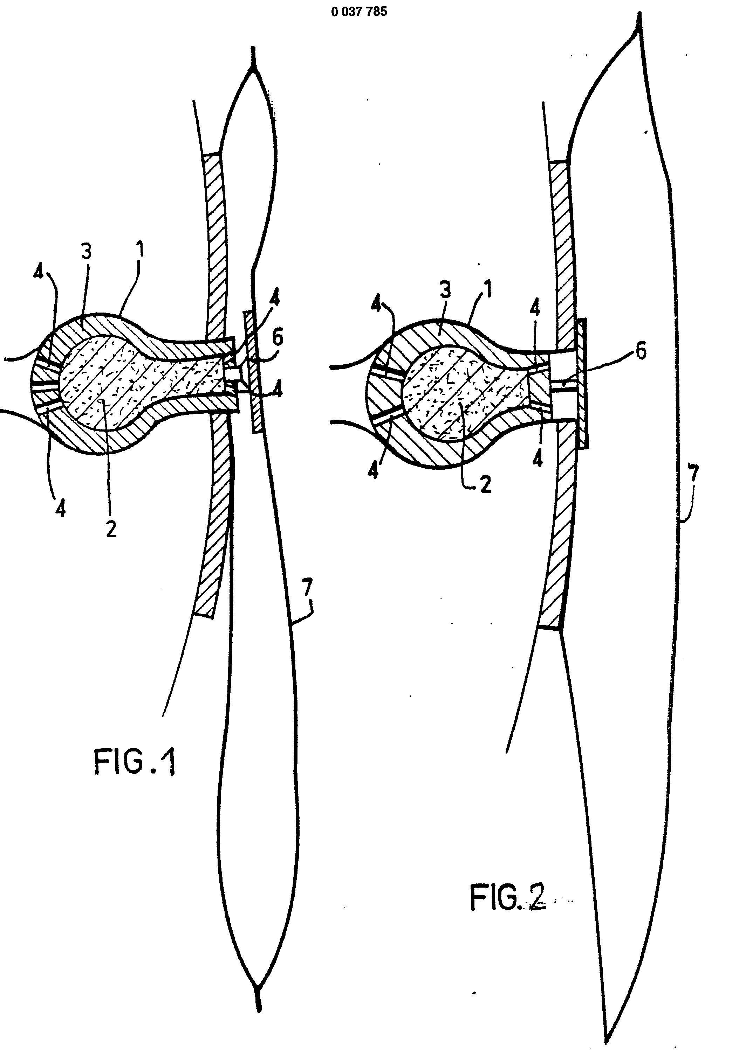

Il faut cependant noter qu'il n'est pas indispensable que ce dispositif, pour être efficace, ait une forme cylindrique parfaite. Au contraire, on peut préférer à un dispositif de forme cylindrique un dispositif sensiblement cylindrique ayant au moins une de ses extrémités arrondies, un dispositif sensiblement piriforme tel que celui de la figure 1, un dispositif sensiblement tronconique ayant sa grande base raccordée par un arrondi au reste du corps, etc.

L'homme de l'art est à même de choisir et de sélectionner, parmi tous les matériaux et matières existants, ceux qui permettent la réalisation du dispositif selon l'invention.

Le corps poreux à cellules ouvertes (2) peut être réalisé en tout matériau de ce type, comme par exemple des mousses de polymères ou d'élastomères, à cellules ouvertes, des feutres ou des tissus, mais on préfère tout particulièrement les mousses de polyuréthane à cellules ouvertes.

Quant à la matière de l'enveloppe (3) de ce corps poreux, ce peut être une matière quelconque assurant un bon glissement sur les parois intestinales et en particulier un caoutchouc naturel ou synthétique du silicone, ou plus généralement tout élastomère souple, mais on peut également réaliser cette enveloppe en un gel élastique essentiellement élaboré à partir de gélatine, de glycérine et d'eau, et qui présente les avantages d'être bio-compatible et bio-dégradable.

Le dispositif selon l'invention comprend à ses deux extrémités, pas nécessairement dans l'axe du corps oblong, mais malgré tout en des endroits qui ne doivent pas être en contact direct avec les parois intestinales, des ouvertures, par exemple des perforations ou des fentes (4), de dimensions et en nombre appropriés. En pratique, des perforations d'environ 1 à 4 mm de diamètre, ou même des fentes dont la plus grande dimension est d'environ 1 à 4 mm, sont tout à fait appropriées.

Plus le nombre des ouvertures est élevé, moins la taille de celles-ci a besoin d'être importante.

Selon la variante schématisée sur la figure 1, cependant, on a pourvu le corps oblong susdit, à son extrémité aval, d'un organe dur ou semi-souple (6) formant une tige dont l'extrémité opposée au corps oblong est évasée en un disque fixé sur la face interne d'une poche de recueil (7) opposée à l'ouverture que celle-ci comporte et qui vient s'appliquer sur l'abouchement de la stomie, et en regard de cette ouverture, de telle sorte que le dispositif éjecté dans ladite poche après une émission de selles puisse être, si on le désire, réinséré manuellement dans la partie terminale de l'intestin, d'où il sera à nouveau éjecté lors de l'émission de selles suivantes.

Dans la variante illustrée schématiquement sur la figure 2, qui s'apparente à celle de la figure 1, l'organe dur ou semi-souple (6), au lieu d'être fixé sur la paroi de la poche de recueil (7) opposée à l'ouverture de celle-ci, est rendu solidaire de la partie frontale interne de la poche de recueil, sur une partie seulement de la périphérie de l'ouverture de celle-ci, inférieure au demi-périmètre de cette ouverture et située de préférence dans la portion de ladite ouverture qui est la portion supérieure lorsque la poche de recueil est portée par l'utilisateur.

Dans ces deux variantes, la fixation de l'organe (6) susdit d'une part au corps oblong (1) et d'autre part à la face interne concernée de la poche de recueil (7) peut être assurée par tout moyen approprié connu de l'homme de l'art, et en particulier par collage ou par soudure.

Chacune des variantes susdites peut encore être améliorée, si on le souhaite, soit par l'introduction dans les cellules ouvertes du matériau poreux (2) d'une charge de matière désodorisante comme par exemple du charbon actif, cette charge de matière absorbant les odeurs pouvant être simplement déposée dans certaines au moins des cellules du matériau poreux, ou être fixées sur leurs parois, par exemple par une résine appropriée, soit par le remplacement d'au moins une partie du matériau poreux (2) par une telle matière absorbante qui, sous forme de fibres, granulés ou autres, constitue en elle-même la matière poreuse.

L'invention est illustrée plus concrètement dans l'exemple de réalisation ci-après, qui ne la limite aucunement.

## EXEMPLE

On réalise un corps oblong ayant un diamètre extérieur de 12 mm et une longueur maximale de 20 mm, en enfermant une mousse de polyuréthane de forme et dimensions appropriées dans une capsule réalisée au moyen d'un gel élastique obtenu après chauffage à 120 °C pendant 15 minutes d'un mélange de 50 % en poids d'eau, 25 % en poids de gélatine et 25 % en poids de glycérine, et refroidissement, et dans laquelle on a ménagé à l'une des extrémités arrondies trois perforations excentriques de chacune 1,5 mm de diamètre et à l'autre une perforation de 4 mm de diamètre, sensiblement dans l'axe de celui-ci, ces perforations faisant communiquer le matériau poreux à cellules ouvertes avec l'extérieur. En outre, ce dispositif est pourvu à l'extrémité portant les trois perforations d'un organe sensiblement en forme de tige dont l'autre extrémité est évasée et fixée par collage sur la paroi interne d'une poche de recueil pour entérostomisés et en regard de l'ouverture dont est munie cette poche.

Ce dispositif a été expérimenté: on a introduit dans l'extrémité de l'intestin d'un malade entérostomisé le corps oblong et placé la poche de recueil. On a pu constater la disparition des émissions sonores que l'on pouvait par contre constater, chez le même malade, à des intervalles irréguliers, lorsque celui-ci portait une poche de recueil de même type, mais ne comportant pas de corps oblong selon l'invention; en outre ce dispositif présente l'avantage de pouvoir être réintroduit manuellement dans la stomie après émission de selles.

## Revendications

1. Poche de recueil (7) pour entérostomisés, caractérisée en ce qu'elle comporte un dispositif atténuateur des bruits, mobile, composé d'un corps oblong (1) en matériau poreux (2), à cellules ouvertes, ledit corps oblong étant gainé par une enveloppe (3) en un matériau n'adhérant pas aux parois de la stomie, l'enveloppe (3) comportant à chaque extrémité au moins une ouverture (4) apte à laisser passer les gaz émis, et pourvu à l'extrémité vers la poche d'un organe dur ou semi-souple (6), notamment une tige, qui se termine en un disque qui est soit fixé sur la face de la poche de recueil, opposée à l'ouverture de la face de la poche qui se fixe autour de la stomie, le disque étant disposé à l'intérieur de la poche, soit rendu solidaire par une partie de sa surface de la face de la poche de recueil portant l'ouverture, le disque étant disposé à l'intérieur de la poche, dans ce dernier cas la zone de fixation étant sur la périphérie de l'ouverture de la poche, cette zone étant inférieure au demi-périmètre de l'ouverture, ladite zone étant située de préférence dans la partie au-dessus de l'ouverture, lorsque la poche de recueil est portée par l'utilisateur.

2. Dispositif selon la revendication 1, caractérisé en ce que le matériau poreux (2) est de la mousse de polyuréthane à cellules ouvertes.

3. Dispositif selon l'une des revendications 1 ou 2, caractérisé en ce que l'enveloppe (3) du corps poreux est en élastomère souple, notamment en caoutchouc naturel ou synthétique.

4. Dispositif selon l'une quelconque des revendications 1 à 3, caractérisé en ce que l'enveloppe (3) du corps poreux est en gel élastique préparé par exemple à partir de gélatine, de glycérine et d'eau.

5. Dispositif selon l'une quelconque des revendications 1 à 4, caractérisé en ce que les ouvertures (4) sont des perforations ou des fentes ayant leur plus grande dimension comprise entre 1 et 4 mm environ.

## Claims

1. A collecting bag (7) for people having enterostomas, characterized in that it comprises a device for reducing noises, detachable comprising an oblong body (1) of open-cell porous material (2), said body being sheathed in an outer casing (3) made of a material that does not adhere to stoma walls, said casing (3) comprising on each end at least an opening (4) suited for passing gases, and having at the end by said bag, a hard or partly flexible mean (6), especially a stem, which is ending in a disk that is either fixed on the collecting bag sheet, opposed to the collecting bag sheet opening which will be placed around the stoma, said disk being inside the bag, or bound through a portion of its surface to the collecting bag sheet containing the opening, said disk being inside the bag in this last case the binding area being at the periphery of the bag opening, said area being smaller than the opening half-perimeter, said area being, preferentially, in the part above the opening, when the collecting bag is used by the patient.

2. Device as claimed in claim 1, characterized in that the porous material (2) is open-cell polyurethane.

3. Device as claimed in claims 1 or 2, characterized in that the porous body casing (3) is made of flexible elastomeric compound, especially of natural or synthetic rubber.

4. Device as claimed in any claim 1 to 3 characterized in that the porous body casing (3) is made of elastic gel, produced for instance from gelatin, glycerine and water.

5. Device as claimed in any claim 1 to 4, characterized in that the openings (4) are perforations or slits, the largest size of which being from about 1 to 4 mm.

## Patentansprüche

1. Auffangtasche (7) für Personen mit künstlichem Darmausgang, dadurch gekennzeichnet, dass sie eine abnehmbare Geräuschdämpfungsvorrichtung umfasst, die aus einem länglichen Körper (1) aus offenzelligem, porösem Material (2) besteht, dass der längliche Körper von einer Umhüllung (3) aus einem an den Wänden des Darmausgangs nicht haftenden Material umgeben ist, dass die Umhüllung (3) an jedem Ende wenigstens eine zum Durchlassen der abgegebenen Körpergase fähige Öffnung (4) aufweist und an dem der Tasche zugekehrten Ende mit einem harten oder halbweichen Organ (6), insbesondere einem Stiel, versehen ist, das bzw. der an einer Scheibe endet, die an derjenigen Seite der Auffangtasche befestigt ist, die der Öffnung in der am Darmausgang festzusetzenden Taschenseite gegenüberliegt, oder dass die Scheibe im Innern der Tasche in fester Verbindung mit einem Bereich der Oberfläche der die Öffnung aufweisenden Auffangtaschenseite angeordnet ist, dass die Scheibe im letzten Fall im Innern der Tasche in einer Befestigungszone angebracht ist, die sich am Umfang der Taschenöffnung befindet, dass diese Zone kleiner ist als der halbe Umfang der Öffnung und dass die genannte Zone vorzugsweise in einem Bereich oberhalb der Öffnung gelegen ist, wenn die Auffangtasche von einem Benutzer getragen wird.

2. Vorrichtung nach Anspruch 1, dadurch gekennzeichnet, dass das poröse Material (2) ein offenzelliger Schaum aus Polyurethan ist.

3. Vorrichtung nach irgendeinem der Ansprüche 1 und 2, dadurch gekennzeichnet, dass die Umhüllung (3) des porösen Körpers ein weiches Elastomer ist, insbesondere ein natürlicher oder synthetischer Kautschuk.

4. Vorrichtung nach irgendeinem der Ansprüche 1 bis 3, dadurch gekennzeichnet, dass die Umhüllung (3) des porösen Körpers ein elastisches Gel ist, das beispielsweise aus Gelatine, Glycerin und Wasser zubereitet ist.

5. Vorrichtung nach irgendeinem der Ansprüche 1 bis 4, dadurch gekennzeichnet, dass die Öffnungen (4) aus Perforationen oder Spalten bestehen, deren grössere Abmessung ungefähr zwischen 1 und 4 mm beträgt.

FIG.1

FIG.2